(19) Europäisches Patentamt / European Patent Office / Office européen des brevets

(11) **EP 2 376 062 B1**

(12) **EUROPEAN PATENT SPECIFICATION**

(45) Date of publication and mention
of the grant of the patent:
**26.04.2017 Bulletin 2017/17**

(21) Application number: **09835146.3**

(22) Date of filing: **29.06.2009**

(51) Int Cl.:
**A61K 9/107** $^{(2006.01)}$     **A61K 9/51** $^{(2006.01)}$

(86) International application number:
**PCT/KR2009/003522**

(87) International publication number:
**WO 2010/074380 (01.07.2010 Gazette 2010/26)**

(54) **PREPARATION METHOD OF POLYMERIC MICELLAR NANOPARTICLES COMPOSITION CONTAINING A POORLY WATER-SOLUBLE DRUG**

VERFAHREN ZUR HERSTELLUNG EINER EIN SCHWER WASSERLÖSLICHES ARZNEIMITTEL ENTHALTENDEN ZUSAMMENSETZUNG VON MIZELLAREN POLYMER-NANOTEILCHEN

MÉTHODE DE PRÉPARATION D'UNE COMPOSITION DE NANOPARTICULES MICELLAIRES POLYMÈRES CONTENANT UN MÉDICAMENT DIFFICILEMENT SOLUBLE DANS L'EAU

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **26.12.2008 KR 20080134539**

(43) Date of publication of application:
**19.10.2011 Bulletin 2011/42**

(73) Proprietor: **Samyang Biopharmaceuticals Corporation**
**Seoul 110-470 (KR)**

(72) Inventors:
• **SEO, Min Hyo**
  **Daejeon 302-782 (KR)**
• **LEE, Sa Won**
  **Daejeon 305-770 (KR)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
EP-A1- 0 877 033     WO-A1-01/12718
WO-A1-03/033592     US-A1- 2004 056 372
US-A1- 2005 226 932     US-B1- 6 210 717

## Description

### Technical Field

This disclosure relates to a method for preparing a poorly water-soluble drug-containing polymeric micellar nanoparticle composition.

### Background Art

Submicronic particulate drug delivery systems using biodegradable polymers have been studied for the purpose of intravenous administration of drugs. Recently, it has been reported that nanoparticle systems and polymeric micelle systems using biodegradable polymers are useful technological systems that modify the *in vivo* distribution of a drug administrated through a vein to reduce undesired side effects and to provide improved efficiency. Additionally, because such systems enable targeted drug delivery, they achieve controlled drug release to a target organ, tissue or cell. In fact, such systems are known to have excellent compatibility with body fluids and to improve the solubilization ability of a poorly water-soluble drug and the bioavailability of a drug.

Recently, there has been reported a method for preparing block copolymer micelles by bonding a drug chemically to a block copolymer containing a hydrophilic segment and a hydrophobic segment. The block copolymer is an A-B type diblock copolymer polymerized from a hydrophilic segment (A) and a hydrophobic segment (B). In the block copolymer, polyethylene oxide is used as a hydrophilic segment (A) and polyaminoacid or hydrophobic group-bound polyaminoacid is used as a hydrophobic segment (B). Such drugs as Adriamycin or Indomethacin may be physically encapsulated within the cores of the polymeric micelles formed from the block copolymer, so that the block copolymer micelles may be used as drug delivery systems. However, the polymeric micelles formed from the block copolymer cause many problems in the case of *in vivo* applications, since they cannot be hydrolyzed but are decomposed merely by enzymes *in vivo,* and they have poor biocompatibility by causing immune responses, or the like.

Therefore, many attempts have been made to develop core-shell type drug delivery systems having improved biodegradability and biocompatibility.

For example, diblock or multiblock copolymers including polyalkylene glycol as a hydrophilic polymer and polylactic acid as a hydrophobic polymer are known to those skilled in the art. More particularly, acrylic acid derivatives are bonded to the end groups of such diblock or multiblock copolymers to form copolymers. The resultant copolymers are subjected to crosslinking to stabilize the polymeric micelles.

However, methods for preparing such diblock or multiblock copolymers have difficulties in introducing crosslinkers to the hydrophobic segments of A-B or A-B-A type diblock or triblock copolymers so that the polymers are in stable structures via crosslinking. Additionally, the crosslinkers used in the above methods cannot ensure safety in the human body because the crosslinkers have no application examples in the human body. Furthermore, the crosslinked polymers cannot be decomposed *in vivo,* and thus cannot be applied to *in vivo* use.

In addition to the above, known methods for preparing a polymeric nanoparticle composition include an emulsification process, a dialysis process and a solvent evaporation process. The emulsification process includes dissolving a biocompatible water-insoluble polymer, such as polylactic acid, into a water immiscible solvent (e.g. methylene chloride or other chlorinated, aliphatic or aromatic solvents), adding a drug to the polymer solution so that the drug is completely dissolved therein, and further adding a surfactant thereto to form an oil-in-water emulsion using a suitable system (e.g. a high-pressure emulsification system or ultrasonic system), and evaporating the emulsion gradually under vacuum. Since the emulsification process requires an equipment for forming the emulsion, it is difficult and sophisticated to set the processing conditions. Additionally, since the emulsification process includes evaporation of an organic solvent, it requires a long period of processing time. Meanwhile, the dialysis process requires consumption of a large amount of water and needs a long period of processing time. Further, the solvent evaporation process requires an equipment, such as a rotary reduced-pressure distillator, for removing a solvent, and it takes a long period of time to remove the solvent completely. Moreover, the solvent evaporation process essentially includes an operation of exposing reagents to a high temperature for a long period of time, and thus it may cause such problems as decomposition of pharmaceutically active ingredients or degradation of pharmacological effects.

WO 03/033592 discloses a process for preparing a polymeric composition containing a poorly water-soluble drug, comprising the steps of dissolving an amphiphilic block copolymer, a polylactic acid derivative, and a poorly water-soluble drug in an organic solvent, evaporating the organic solvent, and adding an aqueous solution to form the poorly water-soluble drug-containing polymeric micelles.

## Disclosure of Invention

### Technical Problem

Provided is a method for preparing a drug-containing polymeric micellar nanoparticle composition.

### Technical Solution

Disclosed herein is a method for preparing a poorly water-soluble drug-containing polymeric micellar nanoparticle composition, which includes: dissolving a poorly water-soluble drug, a salt of polylactic acid or polylactic acid derivative, whose carboxylic acid end is bound to an alkali metal ion, and an amphiphilic block copolymer into an organic solvent; adding an aqueous solution to the resultant mixture in the organic solvent to form micelles; and adding a lyophilization aid to preform lyophilization, wherein the method requires no separate operation to remove the organic solvent prior to the formation of micelles, wherein the polylactic acid or polylactic acid derivative is at least one selected from the group consisting of polylactic acid, polylactide, polyglycolide, polymandelic acid, polycaprolactone, polydioxane-2-one, polyaminoacid, polyorthoester, polyanhydride, and copolymers thereof, wherein the organic solvent is used in an amount of 0.5-15wt% based on the total weight of the composition, and wherein the adding an aqueous solution to the resultant mixture in an organic solvent to form micelles is carried out at 0°C-50°C.

### Advantageous Effects

The method for preparing a poorly water-soluble drug-containing polymeric micellar nanoparticle composition disclosed herein is simple, reduces the processing time, and is amenable to mass production. In addition, the method allows preparation of a poorly water-soluble drug-containing polymeric micellar nanoparticle composition at low temperature or room temperature, thereby improving the stability of a drug.

### Mode for the Invention

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In one aspect, there is provided a method for preparing a drug-containing polymeric micellar nanoparticle composition, which includes:

dissolving a poorly water-soluble drug, a salt of polylactic acid or polylactic acid derivative, whose carboxylic acid end is bound to an alkali metal ion, and an amphiphilic block copolymer into an organic solvent;
adding an aqueous solution to the resultant mixture in the organic solvent to form micelles and
adding a lyophilization aid to perform lyophilization, wherein the method requires no separate operation to remove the organic solvent prior to the formation of micelles,
wherein the polylactic acid or polylactic acid derivative is at least one selected from the group consisting of polylactic acid, polylactide, polyglycolide, polymandelic acid, polycaprolactone, polydioxane-2-one, polyaminoacid, polyorthoester, polyanhydride, and copolymers thereof,

wherein the organic solvent is used in an amount of 0.5-15wt% based on the total weight of the composition, and wherein the adding an aqueous solution to the resultant mixture in an organic solvent to form micelles is carried out at 0°C-50°C.

More particularly, according to the method for preparing a drug-containing polymeric micellar nanoparticle composition disclosed herein, a drug and a polymer are dissolved in a water miscible organic solvent, and then an aqueous solution is added thereto to form polymeric micelles in the mixed organic solvent/water. Therefore, the polymeric micellar nanoparticle composition obtained from the method disclosed herein includes a drug, a salt of polylactic acid or polylactic acid derivative, whose carboxylic acid end is bound to an alkali metal ion, and an amphiphilic block copolymer. In addition, the method requires no separate operation to remove the organic solvent used for the preparation prior to the formation of micelles.

In one embodiment of the method disclosed herein, the method for preparing a drug-containing micellar nanoparticle composition may further include adding divalent or trivalent metal ions after forming the micelles.

The micellar nanoparticles of the invention have diameters of about 10-1000 nanometers, with most of the particles having diameters of under 100 nanometers. This small particle size allows passage through a 0.2 micron filter.

The method for preparing a drug-containing micellar nanoparticle composition includes adding a lyophilization aid to perform lyophilization after forming the micelles.

The presence of an organic solvent in a micelle solution during the formation of micelles facilitates de-association of micelles due to a high affinity of the hydrophobic portion of the amphiphilic polymer micelles to the organic solvent, thereby accelerating precipitation of hydrophobic drug molecules. For this reason, processes for preparing polymeric micelles known to date include dissolving a drug and a polymer into an organic solvent, removing the organic solvent, and adding an aqueous solution thereto to form micelles. However, such processes need a long period of processing time to remove the organic solvent, and require an additional equipment, such as a distillator under reduced pressure. In addition, the organic solvent may still remain partially in the reaction system even after removing it. Further, the drug may be decomposed as it is exposed to high temperature for a long time during the removal of the organic solvent.

According to the invention, micelles are formed at low temperature instead of removing the organic solvent at high temperature during the formation of micelles. In general, when polymeric micelles are heated, associated amphiphilic polymers become susceptible to de-association as the unimer of the amphiphilic polymer get an increased kinetic energy. As a result, hydrophobic drug molecules present in the hydrophobic core of micelles are in contact easily with the aqueous phase, thereby causing formation and precipitation of drug crystals. On the contrary, the method disclosed herein requires no separate solvent evaporation, thereby simplifying the overall process and preventing the decomposition of a drug. Further, the method disclosed herein is carried out at low temperature so that the resultant polymeric micelles maintain their stability.

The polymer micelles are formed by adding an aqueous solution to the drug/amphiphilic polymer mixture in an organic solvent at a temperature of 0-50°C, more particularly 0-40°C.

However, even though the organic solvent is not removed but exists at a certain concentration or higher as in the method disclosed herein, forming micelles while maintaining low temperature may prevent precipitation of a drug. This is because the polymer and organic solvent molecules have a decreased dynamic energy under such a low temperature, and thus the drug present in the hydrophobic segment of the polymeric micelles may not be easily exposed to the aqueous phase.

In one embodiment, the drug may be selected from poorly water-soluble drugs having a solubility of 100 mg/mL or less to water.

In still another embodiment, the poorly water-soluble drug may be selected from anticancer agents. Particularly, the poorly water-soluble drug may be selected from taxane anticancer agents. Particular examples of the taxane anticancer agents may include paclitaxel, docetaxel, 7-epipaclitaxel, t-acetyl paclitaxel, 10-desacetyl-paclitaxel, 10-desacetyl-7-epipaclitaxel, 7-xylosylpaclitaxel, 10-desacetyl-7-glutarylpaclitaxel, 7-N,N-dimethylglycylpaclitaxel, 7-L-alanylpaclitaxel or a mixture thereof. More particularly, the taxane anticancer agent may be paclitaxel or docetaxel.

In one embodiment of the process, the amphiphilic block copolymer includes a diblock copolymer having a hydrophilic block (A) and a hydrophobic block (B) linked with each other in the form of A-B structure, and is non-ionic. Additionally, the amphiphilic block copolymer forms core-shell type polymeric micelles in the aqueous enviroment, wherein the hydrophobic block (B) forms the core and the hydrophilic block (A) forms the shell.

In another embodiment of the process, the hydrophilic block (A) of the amphiphilic block copolymer is a water soluble polymer, and includes at least one selected from the group consisting of polyalkylene glycol, polyvinyl alcohol, polyvinyl pyrrolidone, polyacrylamide and derivatives thereof. Particularly, the hydrophilic block (A) may be at least one selected from the group consisting of polyalkylene glycol, monomethoxypolyalkylene glycol, monoacetoxypolyalkylene glycol, polyethylene-co-propylene glycol, and polyvinyl pyrrolidone. More particularly, the hydrophilic block (A) may be at least one selected from the group consisting of polyethylene glycol, monomethoxypolyethylene glycol, monoacetoxypolyethylene glycol, and polyethylene-co-propylene glycol.

In addition, the hydrophilic block (A) used in the method may have a number average molecular weight of 500-50,000 daltons, particularly 1,000-20,000 daltons, and more particularly 1,000-10,000 daltons.

The hydrophobic block (B) of the amphiphilic block copolymer is not dissolved in water and may be a biodegradable polymer with high biocompatibility. For example, the hydrophobic block (B) may be at least one polymer selected from the group consisting of polyester, polyanhydride, polyamino acid, polyorthoester, polyphosphazine, etc. More particularly, the hydrophobic block (B) may be selected from the group consisting of polylactide, polyglycolide, polycaprolactone, polydioxane-2-one, polylactic-*co*-glycolide, polylactic-*co*-dioxane-2-one, polylactic-*co*-caprolactone and polyglycolic-*co*-caprolactone. In addition, the hydroxyl end groups of the hydrophobic block (B) may be substituted with fatty acid groups. The fatty acid group may be at least one selected from the group consisting of butyrate, propionate, acetate, stearate, palmitate, cholesterol group, and tocopherol group.

Meanwhile, the hydrophobic block (B) of the amphiphilic block copolymer may have a number average molecular weight of 500-50,000 daltons, particularly 1,000-20,000 daltons, and more particularly 1,000-10,000 daltons.

In still another embodiment, to form stable polymeric micelles in an aqueous solution, the amphiphilic block copolymer includes the hydrophilic block (A) and the hydrophobic block (B) in a weight ratio of 3:7 to 8:2 (hydrophilic block (A) : hydrophobic block (B)), particularly of 4:6 to 7:3. When the proportion of the hydrophilic block (A) is lower than the above range, the polymer may not form polymeric micelles in an aqueous solution. On the other hand, the proportion of the hydrophilic block (A) is higher than the above range, the polymer is too hydrophilic to maintain its stability.

In addition, in the salt of polylactic acid or polylactic acid derivative, whose carboxylic acid end is bound to an alkali metal ion, the polylactic acid or polylactic acid is at least one selected from the group consisting of polylactic acid, polylactide, polyglycolide, polymandelic acid, polycaprolactone, polydioxane-2-one, polyamino acid, polyorthoester, polyanhydride, and copolymers thereof. Particularly, the polylactic acid or polylactic acid derivative is polylactic acid, polylactide, polyglycolide, polycaprolactone or polydioxane-2-one. More particularly, the polylactic acid or polylactic acid derivative may be at least one selected from the group consisting of polylactic acid, polylactide, polycaprolactone, polylactic-*co*-mandelic acid, polylactic-*co*-glycolide, polylactic-*co*-caprolactone, and polylactic-*co* - 1,4-dioxane-2-one.

In one embodiment, the salt of polylactic acid or polylactic acid derivative, whose carboxylic acid end is bound to an alkali metal ion, includes at least one carboxylate salt at one end, and at least one selected from the group consisting of hydroxy, acetoxy, benzoyloxy, decanoyloxy, palmitoyloxy and alkoxy at the other end. In addition, the carboxylate salt functions as a hydrophilic group in an aqueous solution with a pH 4 or higher, thereby forming polymeric micelles in an aqueous solution.

In one embodiment, the alkali metal ion may be a monovalent metal ion such as sodium, potassium or lithium. In addition, the salt of polylactic acid or polylactic acid derivative is present in a solid state at room temperature, and is very stable even when exposed to moisture in the air because of its neutral pH in an aqueous solution.

The salt of polylactic acid or polylactic acid derivative, whose carboxylic acid end is bound to an alkali metal ion, is added to the micelles formed of the amphiphilic block copolymer to harden the inner parts of the micelles, and thus improves the drug encapsulation efficiency within the micelles. When the salt of polylactic acid or polylactic acid derivative is dissolved in an aqueous solution, the hydrophilic segment and the hydrophobic segment present in the molecule are balanced with each other to form micelles. Therefore, when the hydrophobic ester segments have an increased molecular weight, the hydrophilic terminal carboxylic acid anions are hardly associated, making it difficult to form micelles. On the other hand, when the hydrophobic ester segments have an excessively low molecular weight, the salt of the polymer is dissolved completely in water, making it difficult to form micelles. For example, the polylactic acid or polylactic acid derivative capable of forming micelles at pH 4 or higher may have a number average molecular weight of 500-5,000, specifically 500-2,500. If the molecular weight is less than 500 daltons, the salt of polylactic acid or polylactic acid derivative is dissolved completely in water, making it difficult to form micelles. If the molecular weight is greater than 5,000 daltons, the salt of polylactic acid or polylactic acid derivative has excessively high hydrophobicity and is hardly soluble in an aqueous solution, thereby hindering micelle formation. The molecular weight of the polylactic acid or polylactic acid derivative may be controlled by adjusting the reaction temperature and time, etc. during the preparation thereof.

For example, the organic solvent used in the method may be a water miscible organic solvent, and may be at least one solvent selected from the group consisting of alcohol, acetone, tetrahydrofuran, acetic acid, acetonitrile and dioxane. Although the organic solvent is required to dissolve the polymer and the drug, the organic solvent may be used in the method in a small amount, because the presence of the organic solvent decreases the micelle stability and accelerates drug precipitation. The organic solvent is used in an amount of 0.5-15 wt%, and more particularly 1-10 wt%, based on the total weight of the polymeric micellar nanoparticle composition. When the organic solvent is used in an amount less than 0.5 wt%, it is difficult to dissolve the drug in the organic solvent. On the other hand, when the organic solvent would be used in an amount greater than 30 wt%, drug precipitation would occur during the reconstitution.

The poorly water-soluble drug may be dissolved into the organic solvent sequentially or simultaneously with the polymer.

In the method disclosed herein, to dissolve the poorly water-soluble drug, the salt of polylactic acid or polylactic acid derivative, and the amphiphilic block copolymer into the organic solvent, the drug and the polymer may be simultaneously added to and dissolved into the organic solvent. Otherwise, the polymer may be dissolved first into the organic solvent, followed by the drug, or vice versa. The poorly water-soluble drug, the salt of polylactic acid or polylactic acid derivative, and the amphiphilic block copolymer may be dissolved into the organic solvent at any temperature where the drug decomposition is prevented. As a non-limiting example, the temperature may be 0-60°C, particularly 0-50°C, and more particularly 0-40°C.

A particular embodiment of the method for preparing a drug-containing polymeric micellar nanoparticle composition includes:

dissolving a salt of polylactic acid or polylactic acid derivative, whose carboxylic acid end is bound to an alkali metal ion, and an amphiphilic block copolymer into an organic solvent;
dissolving a poorly water-soluble drug into the resultant polymeric solution in the organic solvent; and
adding an aqueous solution to the resultant solution of the poorly water-soluble drug in the polymeric solution to form polymeric micelles,
wherein the method for preparing a drug-containing polymeric micellar nanoparticle composition requires no separate operation to remove the organic solvent prior to the formation of micelles.
The aqueous solution used in the method may include water, distilled water, distilled water for injection, saline, 5% glucose, buffer, etc.

The polymeric micelle formation is carried out by adding the aqueous solution at a temperature of 0-50°C, and more particularly 0-40°C.

In one embodiment, the method for preparing a drug-containing micellar nanoparticle composition may further include adding divalent or trivalent metal ions after forming the micelles. The divalent or trivalent metal ion may be selected from the group consisting of calcium ($Ca^{2+}$), magnesium ($Mg^{2+}$), barium ($Ba^{2+}$), chrome ($Cr^{3+}$), iron ($Fe^{3+}$), manganese ($Mn^{2+}$), nickel ($Ni^{2+}$), copper ($Cu^{2+}$), zinc ($Zn^{2+}$) and aluminum ($Al^{3+}$). In another embodiment, the divalent or trivalent metal ions may be added to the polymeric composition including the amphiphilic block copolymer mixed with the salt of polylactic acid or polylactic acid derivative in the form of sulfate, hydrochloride, carbonate, phosphate and hydroxide. More particularly, the divalent or trivalent metal ions may be added in the form of calcium chloride ($CaCl_2$), magnesium chloride ($MgCl_2$), zinc chloride ($ZnCl_2$), aluminum chloride ($AlCl_3$), ferric chloride ($FeCl_3$), calcium carbonate ($CaCO_3$), magnesium carbonate ($MgCO_3$), calcium phosphate ($Ca_3(PO_4)_2$), magnesium phosphate ($Mg_3(PO_4)_2$), aluminum phosphate ($AlPO_4$), magnesium sulfate ($MgSO_4$), calcium hydroxide ($Ca(OH)_2$), magnesium hydroxide ($Mg(OH)_2$), aluminum hydroxide ($Al(OH)_3$) and zinc hydroxide ($Zn(OH)_2$).

In addition, the divalent or trivalent metal ions may be used in an amount of 0.001-10 equivalents, particularly 0.5-2.0 equivalents, based on the total equivalent of the carboxyl end groups of the salt of polylactic acid or polylactic acid derivative.

In a particular embodiment, the divalent or trivalent metal ions may be added to further improve the stability of polymeric micelles formed by mixing the amphiphilic block copolymer and the salt of polylactic acid or polylactic acid derivative. The divalent or trivalent metal ions are bound to the terminal carboxyl groups of the polylactic acid or polylactic acid derivative to form polymeric micellar nanoparticles to which the divalent or trivalent metal ions are bound. The divalent or trivalent metal ions are subjected to a substitution reaction with the monovalent metal cations at the polylactic acid carboxyl end groups in the polymeric micelles, thereby forming ionic bonds. The resultant ionic bonds formed by the metal ions serve to further improve the stability of the polymeric micelles by virtue of the strong binding force.

A lyophilization aid is added to the micelle composition to perform lyophilization, after forming the polymeric micelles. Particularly, the lyophilization aid may be at least one selected from the group consisting of sugar, sugar alcohol, and mixtures thereof. The sugar may be at least one selected from the group consisting of lactose, maltose, sucrose, trehalose and a combination thereof. The sugar alcohol may be at least one selected from the group consisting of mannitol, sorbitol, maltitol, xylitol, lactitol and a combination thereof. The lyophilization aid may be added in order to allow a lyophilized composition to maintain its cake-like shape. In addition, the lyophilization aid serves to help the polymeric micellar nanoparticle composition to be dissolved homogeneously in a short time during the reconstitution of the lyophilized polymeric micellar nanoparticle composition. In this context, the lyophilization aid may be used in an amount of 1-90 wt%, and more particularly 10-60 wt%, based on the total weight of the lyophilized composition.

In the poorly water-soluble drug-containing polymeric micellar nanoparticle composition, the poorly water-soluble drug is used in a weight ratio of 0.1-50.0:50.0-99.9, particularly 0.1-20.0:80.0-99.9 based on the combined weight of the amphiphilic block copolymer and the salt of polylactic acid or polylactic acid derivative. The poorly water-soluble drug-containing polymeric micellar nanoparticle composition may include, based on the total weight including the amphiphilic block copolymer and the salt of polylactic acid or polylactic acid derivative, 0.1-99.9 wt% of the amphiphilic block copolymer and 0.1-99.9 wt% of the salt of polylactic acid or polylactic acid derivative. Particularly, the polymeric micellar nanoparticle composition may include 20-95 wt% of the amphiphilic block copolymer and 5-80 wt% of the salt of polylactic acid or polylactic acid derivative. More particularly, the polymeric micellar nanoparticle composition may include 50-90 wt% of the amphiphilic block copolymer and 10-50 wt% of the salt of polylactic acid or polylactic acid derivative.

The method for preparing a poorly water-soluble drug-containing polymeric micellar nanoparticle composition disclosed herein is simple, reduces the processing time, and is amenable to mass production. In addition, the method allows preparation of a poorly water-soluble drug-containing polymeric micellar nanoparticle composition at low temperature or room temperature, thereby improving the stability of a drug.

In still another embodiment, the drug-containing polymeric micellar nanoparticle composition may further include pharmaceutical excipients, such as a preservative, stabilizer, hydrating agent or emulsification accelerator, salt for adjusting osmotic pressure and/or buffer, as well as other therapeutically useful materials. The composition may be formulated into various types of oral or parenteral formulations according to a manner generally known to those skilled in the art.

Formulations for parenteral administration may be administered via a rectal, local, transdermal, intravenous, intramuscular, intraperitoneal, subcutaneous route, etc. Typical examples of the parenteral formulations include injection formulations in the form of an isotonic aqueous solution or suspension. In one example embodiment, the composition may be provided in a lyophilized form, which is to be reconstituted with distilled water for injection, 5% glucose, saline, etc., so that it is administered via intravascular injection.

Formulations for oral administration include tablets, pills, hard and soft capsules, liquid, suspension, emulsion, syrup, granules, etc. Such formulations may include a diluent (e.g. lactose, dextrose, sucrose, mannitol, sorbitol, cellulose and glycine), a glidant (e.g. silica, talc, stearic acid and magnesium or calcium salts thereof, as well as polyethylene glycol), etc. in addition to active ingredients. Tablets may include binders, such as magnesium aluminum silicate, starch paste, gelatin, tragacanth, methyl cellulose, sodium carboxymethyl cellulose and polyvinyl pyrrolidine. Optionally, tablets may include pharmaceutically acceptable additives including disintegrating agents such as starch, agar, alginate or sodium salt thereof, absorbing agents, coloring agents, flavoring agents and sweetening agents. Tablets may be obtained by a conventional mixing, granulating or coating process. In addition, typical examples of formulations for parenteral administration include injection formulations, such as isotonic aqueous solutions or suspensions.

The examples and experiments will now be described. The following examples and experiments are for illustrative purposes only and not intended to limit the scope of this disclosure.

The amphiphilic block copolymer and the salt of polylactic acid or polylactic acid derivative, whose carboxylic acid end is bound to an alkali metal ion, used in the method disclosed herein were obtained according to the method as described in International Patent Publication No. WO 03/033592.

### [Examples 1-3] Preparation of Polymeric Micellar Nanoparticles Containing Docetaxel

As an amphiphilic block copolymer, monomethoxypolyethylene glycol-polylactide having a number average molecular weight of 2,000-1,766 daltons was prepared. D,L-PLA-COONa having a number average molecular weight of 1,800 daltons was also prepared.

The amphiphilic block copolymer and the polylactic acid salt were completely dissolved at 60°C in the amounts as described in Table 1, and 2.5 mL of ethanol was added thereto, followed by thorough mixing. Next, the resultant mixture was cooled to 30°C, docetaxel was added thereto, and the mixture was agitated until a clear solution containing docetaxel completely dissolved therein was obtained. Then, the solution was cooled to 25°C, and 40.0 mL of purified water at room temperature was added thereto, and the reaction mixture was allowed to react until a bluish clear solution was formed, thereby forming micellar nanoparticles. Calcium chloride was further added thereto to form polymeric micellar nanoparticles. Then, D-mannitol as a lyophilizing agent was added to and completely dissolved into the polymeric micellar nanoparticle solution, and the resultant solution was filtered through a filter with a pore size of 200 nm, followed by lyophilization, to obtain a powdery docetaxel-containing polymeric micellar nanoparticle composition.

Table 1

[Table 1]

| | | Amount (mg) | | | | | |
|---|---|---|---|---|---|---|---|
| | | Docetaxel | Polylactic Acid Salt [1] | Amphiphilic Block Copolymer [2] | Metal Ion Salt [3] | Lyophilization Aid[4] | Anhydrous Ethanol | Purified Water |
| Example 1 | | 80.0 | 3,960.0 | 3,960.0 | 244.18 | 916.02 | 1,972.5 (2.50 mL) | 41,673 |

(continued)

|  | Amount (mg) | | | | | | |
|---|---|---|---|---|---|---|---|
|  | Docetaxel | Polylactic Acid Salt [1] | Amphiphilic Block Copolymer [2] | Metal Ion Salt [3] | Lyophilization Aid[4] | Anhydrous Ethanol | Purified Water |
| Example 2 | 80.0 | 2,613.3 | 1,306.7 | 80.57 | 453.40 | 978.4 (1.24 mL) | 20,888 |
| Example 3 | 80.0 | 1,140.0 | 380.0 | 23.43 | 180.38 | 378.7 (0.48 mL) | 8,379 |
| 1) D,L-PLA-COONa, Mn (number average molecular weight) 1,800 daltons 2) Monomethoxypolyethyleneglycol-polylactide, Mn 2,000-1,766 daltons 3) Calcium chloride 4) D-mannitol | | | | | | | |

**[Examples 4-6] Preparation of Polymeric Micellar Nanoparticles Containing Paclitaxel**

As an amphiphilic block copolymer, monomethoxypolyethylene glycol-polylactide having a number average molecular weight of 2,000-1,766 daltons was prepared. D,L-PLA-COONa having a number average molecular weight of 1,800 daltons was also prepared.

The amphiphilic block copolymer and the polylactic acid salt were completely dissolved at 80°C under agitation in the amounts as described in Table 2, and ethanol was added thereto, followed by thorough mixing. Next, paclitaxel was added to the ethanol solution containing the polymer, and the resultant mixture was agitated until a clear solution containing paclitaxel completely dissolved therein was obtained. Then, the solution was cooled to 50°C, and 40.0 mL of purified water at room temperature was added thereto, and the reaction mixture was allowed to react until a bluish clear solution was formed, thereby forming micellar nanoparticles. An aqueous calcium chloride solution was further added thereto to form polymeric micellar nanoparticles containing paclitaxel. Then, D-mannitol as a lyophilizing agent was added to and completely dissolved into the polymeric micellar nanoparticle solution, and the resultant solution was filtered through a filter with a pore size of 200 nm, followed by lyophilization, to obtain a powdery paclitaxel-containing polymeric micellar nanoparticle composition.

Table 2

[Table 2]

|  | Amount (mg) | | | | | | |
|---|---|---|---|---|---|---|---|
|  | Paclitaxel | Polylactic Acid Salt [1] | Amphiphilic Block Copolymer | Metal Ion Salt[3] | Lyophilization Aid[4] | Anhydrous Ethanol | Purified Water |
| Example 4 | 100.0 | 4,950.0 | 4,950.0 | 305.22 | 1,818.6 | 2,461.7 (3.12 mL) | 51,423 |
| Example 5 | 100.0 | 3,266.7 | 1,633.3 | 100.71 | 900.1 | 1,215.1 (1.54 mL) | 25,792 |
| Example 6 | 100.0 | 1,425.0 | 475.0 | 29.29 | 358.1 | 473.4 (0.60 mL) | 10,346 |
| 1) D,L-PLA-COONa, Mn 1,800 daltons 2) Monomethoxypolyethyleneglycol-polylactide, Mn 2,000-1,766 daltons 3) Calcium chloride 4) D-mannitol | | | | | | | |

**[Comparative Example 1] Preparation of Docetaxel-Containing Polymeric Micellar Nanoparticles Using Solvent Evaporation Process**

First, docetaxel, the amphiphilic block copolymer and the polylactic acid salt were provided in the same amounts as described in Example 3. Next, 5 mL of ethanol was added to docetaxel and the polymer, and the resultant mixture was agitated at 60°C until the materials were completely dissolved to obtain a clear solution. Then, ethanol was distilled off under reduced pressure at 60°C for 3 hours using a rotary reduced-pressure distillator equipped with a round bottom flask. The reaction mixture was cooled to 25°C, 4 mL of purified water at room temperature was added thereto and the reaction mixture was allowed to react until a bluish clear solution was obtained, thereby forming polymeric micellar nanoparticles. An aqueous calcium chloride solution was added thereto to form polymeric micellar nanoparticles. Then, 100 mg of D-mannitol as a lyophilizing agent was added to and completely dissolved into the polymeric micellar nanoparticle solution and the resultant mixture was filtered through a filter with a pore size of 200 nm, followed by lyophilization, thereby providing a powdery docetaxel-containing polymeric micellar nanoparticle composition.

**[Comparative Example 2] Preparation of Paclitaxel-Containing Polymeric Micellar Nanoparticles Using Solvent Evaporation Process**

First, paclitaxel, the amphiphilic block copolymer and the polylactic acid salt were provided in the same amounts as described in Example 6. Next, 5 mL of ethanol was added to paclitaxel and the polymer, and the resultant mixture was agitated at 60°C until the materials were completely dissolved to obtain a clear solution. Then, ethanol was distilled off under reduced pressure at 60°C for 3 hours using a rotary reduced-pressure distillator equipped with a round bottom flask. The reaction mixture was cooled to 50°C, 5 mL of purified water at room temperature was added thereto and the reaction mixture was allowed to react until a bluish clear solution was obtained, thereby forming polymeric micellar nanoparticles. An aqueous calcium chloride solution was added thereto to form polymeric micellar nanoparticles. Then, 100 mg of anhydrous lactose as a lyophilizing agent was added to and completely dissolved into the polymeric micellar nanoparticle solution, and the resultant mixture was filtered through a filter with a pore size of 200 nm, followed by lyophilization, thereby providing a powdery paclitaxel-containing polymeric micellar nanoparticle composition.

**[Comparative Example 3] Preparation of Docetaxel-Containing Polymeric Micellar Nanoparticles Using Solvent Evaporation Process**

First, the polylactic acid salt and the amphiphilic block copolymer provided in the same amounts as described in Example 3 of Table 1 were completely dissolved at 60°C, and 5 mL of ethanol was added thereto, followed by thorough mixing. The resultant mixture was cooled to 30°C, docetaxel was added thereto and the mixture was further agitated until a clear solution containing docetaxel completely dissolved therein was obtained. Then, ethanol was distilled off under reduced pressure using a rotary reduced-pressure distillator equipped with a round bottom flask. The reaction mixture was cooled to 25°C, purified water at room temperature was added thereto and the reaction mixture was allowed to react until a bluish clear solution was obtained, thereby forming micellar nanoparticles. An aqueous calcium chloride solution was added thereto to form polymeric micellar nanoparticles. Then, D-mannitol as a lyophilizing agent was added to and completely dissolved into the polymeric micellar nanoparticle solution, and the resultant mixture was filtered through a filter with a pore size of 200 nm, followed by lyophilization, thereby providing a powdery docetaxel-containing polymeric micellar nanoparticle composition.

**[Comparative Example 4] Preparation of Paclitaxel-Containing Polymeric Micellar Nanoparticles Using Solvent Evaporation Process**

First, the amphiphilic block copolymer and the polylactic acid salt provided in the same amounts as described in Example 6 of Table 2 were completely dissolved at 60°C, and 5.0 mL of ethanol was added thereto, followed by thorough mixing. After that, paclitaxel was added thereto and the mixture was further agitated until a clear solution containing paclitaxel completely dissolved therein was obtained. Then, ethanol was distilled off under reduced pressure using a rotary reduced-pressure distillator equipped with a round bottom flask. Purified water at room temperature was added thereto and the reaction mixture was allowed to react until a bluish clear solution was obtained, thereby forming micellar nanoparticles. An aqueous calcium chloride solution was added thereto to form polymeric micellar nanoparticles. Then, D-mannitol as a lyophilizing agent was added to and completely dissolved into the polymeric micellar nanoparticle solution, and the resultant mixture was filtered through a filter with a pore size of 200 nm, followed by lyophilization, thereby providing a powdery paclitaxel-containing polymeric micellar nanoparticle composition.

**[Comparative Example 5] Preparation of Micellar Nanoparticles at High Temperature**

A docetaxel-containing polymeric micellar nanoparticle composition was prepared in the same amount and manner as described in Example 1, except that the polymeric micellar nanoparticles were formed while maintaining the temperature at 70°C after adding the ethanol solution. After that, the micellar nanoparticles were lyophilized in the same manner as described in Example 1 to obtain a lyophilized micellar nanoparticle composition.

**[Comparative Example 6] Preparation of Micellar Nanoparticles at High Temperature**

A paclitaxel-containing polymeric micellar nanoparticle composition was prepared in the same amount and manner as described in Example 6, except that the polymeric micellar nanoparticles were formed while maintaining the temperature at 70°C after adding the ethanol solution. After that, the micellar nanoparticles were lyophilized in the same manner as described in Example 6 to obtain a lyophilized micellar nanoparticle composition.

**[Example 7] Preparation of Docetaxel-Containing Polymeric Micellar Nanoparticles**

First, the amphiphilic block copolymer and the polylactic acid salt provided in the same amounts as described in Example 1 were completely dissolved at 60°C, and 2.5 mL of ethanol was added thereto, followed by thorough mixing. The resultant mixture was cooled to 30°C. After that, docetaxel was added thereto and the mixture was further agitated until a clear solution containing docetaxel completely dissolved therein was obtained. Then, the resultant solution was cooled to 25°C, 40.0 mL of purified water at room temperature was added thereto, and the reaction mixture was allowed to react until a bluish clear solution was obtained, thereby forming micellar nanoparticles. In this example, calcium chloride was not added. Then, D-mannitol as a lyophilizing agent was added to and completely dissolved into the polymeric micelle solution, and the resultant mixture was filtered through a filter with a pore size of 200 nm, followed by lyophilization, thereby providing a powdery docetaxel-containing polymeric micellar nanoparticle composition.

**[Example 8] Preparation of Paclitaxel-Containing Polymeric Micellar Nanoparticles**

First, the amphiphilic block copolymer and the polylactic acid salt provided in the same amounts as described in Example 4 were completely dissolved at 80°C, and ethanol was added thereto, followed by thorough mixing. After that, paclitaxel was added thereto and the mixture was further agitated until a clear solution containing paclitaxel completely dissolved therein was obtained. Then, the resultant solution is cooled to 50°C, 40.0 mL of purified water at room temperature was added thereto, and the reaction mixture was allowed to react until a bluish clear solution was obtained, thereby forming micellar nanoparticles. In this example, calcium chloride was not added. Then, D-mannitol as a lyophilizing agent was added to and completely dissolved into the micelle solution, and the resultant mixture was filtered through a filter with a pore size of 200 nm, followed by lyophilization, thereby providing a powdery paclitaxel-containing polymeric micellar nanoparticle composition.

**[Test Example 1] Measurement of Amount of Drug Encapsulation**

The docetaxel-containing polymeric micellar nanoparticle compositions according to Examples 1-3 and Comparative Examples 1 and 3 were subjected to HPLC as specified in Table 3 to measure the concentration of docetaxel in each composition. Then, the drug content (encapsulation amount) was calculated according to Math Figure 1. The results were shown in Table 4.

[Math Figure 1]

$$\text{Encapsulation (\%)} = (\text{measured amount of docetaxel / amount of used docetaxel}) \times 100$$

Table 3

[Table 3]

|  | Condition |
|---|---|
| Mobile Phase | 45% Acetonitrile/55% Water |
| Column | C18, 300A Inner Diameter 4.6 mm, Length 25 cm (Phenomenex. USA) |
| Detection Wavelength | 227 nm |
| Flow Rate | 1.5 mL/min. |
| Temperature | Room Temperature |

(continued)

| | Condition |
|---|---|
| Injection Volume | 10 μL |

Table 4

[Table 4]

| | Docetaxel Content (%) |
|---|---|
| Example 1 | 104.2 |
| Example 2 | 102.5 |
| Example 3 | 103.2 |
| Comparative Example 1 | 78.5 |
| Comparative Example 3 | 101.7 |

As can be seen from the above results of Table 4, the compositions obtained after lyophilization without removing the organic solvent according to Examples 1-3 show a docetaxel content of about 100%. On the other hand, the lyophilized composition obtained after removing the organic solvent according to Comparative Example 1 shows a docetaxel content of about 78.5%. This demonstrates that docetaxel is decomposed in the polymeric micellar nanoparticles obtained via a solvent evaporation process according to Comparative Example 1 during the evaporation of the organic solvent at high temperature.

In addition, the lyophilized composition obtained after removing the organic solvent at 30°C according to Comparative Example 3 shows a similar docetaxel content. Therefore, it can be seen that the method disclosed herein provides a similar drug encapsulation amount as compared to the conventional solvent evaporation process, while simplifying the overall process by avoiding a need for separate operation of removing the organic solvent.

## [Test Example 2] Measurement of Particle Size

The paclitaxel-containing polymeric micellar nanoparticle compositions according to Examples 4-6 and Comparative Examples 2 and 4 were reconstituted with saline, and the particle size in each reconstituted composition was measured in aqueous solution using a particle size analyzer (DLS). The results were shown in Table 5.

Table 5

[Table 5]

| | Particle Size (nm) |
|---|---|
| Example 4 | 27.5 |
| Example 5 | 26.7 |
| Example 6 | 20.5 |
| Comparative Example 2 | 20.3 |
| Comparative Example 4 | 20.4 |

As can be seen from the above results of Table 5, there is no significant difference in the particle size in aqueous solution between the lyophilized compositions obtained without removing the organic solvent according to Examples 4-6 and the lyophilized compositions obtained after removing the organic solvent according to Comparative Examples 2 and 4.

## [Test Example 3] Stability Test

The paclitaxel-containing polymeric nanoparticle composition according to Example 6 was compared with the paclitaxel-containing polymeric nanoparticle composition according to Comparative Example 2 in terms of the stability in aqueous solution at 37°C.

Each of the compositions according to Example 6 and Comparative Example 2 was diluted with distilled water for injection to a paclitaxel concentration of 1 mg/mL. While each diluted solution was allowed to stand at 37°C, concentration of paclitaxel contained in the nanoparticles was measured over time by way of HPLC. HPLC was carried out under the same conditions as described in Table 3. The results were shown in Table 6.

Table 6

[Table 6]

| Time (hr) | Paclitaxel Concentration (mg/mL) | |
|---|---|---|
| | Example 6 | Comparative Example 2 |
| 0 | 1.00 | 1.00 |
| 2 | 1.00 | 0.99 |
| 4 | 0.99 | 0.99 |
| 8 | 0.99 | 0.99 |
| 12 | 0.99 | 0.98 |
| 24 | 0.99 | 0.98 |

As can be seen from the above results of Table 6, there is no significant difference in the stability in aqueous solution over 24 hours between the lyophilized composition obtained without removing the organic solvent according to Example 6 and the lyophilized composition obtained after removing the organic solvent according to Comparative Example 2.

**[Test Example 4]**

The docetaxel-containing polymeric micellar nanoparticle compositions according to Example 1 and Comparative Example 5 were compared with each other in terms of the docetaxel content and related compound content. The docetaxel content was measured under the same HPLC conditions as described in Table 3, and the related compound content was measured under the same HPLC conditions as described in Table 7. The results were shown in Table 8.

Table 7

[Table 7]

| | Condition | |
|---|---|---|
| Mobile Phase | Time (min.) | Water: Acetonitrile |
| | 0-15 | 65:35→35:65 |
| | 15-25 | 35:65→25:75 |
| | 25-30 | 25:75→5:95 |
| | 30-35 | 5:95→0:100 |
| | 35-39 | 0:100 |
| | 39-40 | 0:100→65:35 |
| | 40-45 | 65:35 |
| Column | C18, 300A Inner Diameter 4.6 mm, Length 25 cm (Phenomenex, USA) | |
| Detection Wavelength | 230 nm | |
| Flow Rate | 1.0 mL/min. | |
| Temperature | Room Temperature | |
| Injection Volume | 10 μL | |

Table 8

[Table 8]

| | Content (%) | |
|---|---|---|
| | Docetaxel | Total related compounds |
| Example 1 | 104.2 | 0.67 |
| Comp. Ex. 5 | 92.1 | 8.73 |

As can be seen from the above results, high-temperature preparation causes an increase in the amount of docetaxel-related compounds to at least 10 times of the amount of those compounds in the case of Example 1, resulting in a drop in the docetaxel content in polymeric micellar nanopraticles. This means that high-temperature processing conditions cause decomposition of a drug.

**[Test Example 5]**

The paclitaxel-containing polymeric micellar nanoparticle composition according to Example 6 was compared with the paclitaxel-containing polymeric micellar nanoparticle composition according to Comparative Example 6 in terms of the paclitaxel content. The paclitaxel content was measured under the same HPLC conditions as described in Table 3. Then, the drug content (encapsulation amount) was calculated according to Math Figure 2. The results were shown in Table 9.

[Math Figure 2]

$$\text{Encapsulation } (\%) = [\text{measured amount of paclitaxel} / \text{amount of used paclitaxel}] \times 100$$

Table 9

[Table 9]

| | Paclitaxel content (%) |
|---|---|
| Example 6 | 99.3 |
| Comp. Ex. 6 | 80.9 |

As can be seen from the above results, the polymeric nanoparticle composition obtained by adding water to form micelles in the presence of the organic solvent while maintaining a high temperature of 70°C according to Comparative Example 6 causes precipitation of the drug, paclitaxel. After the sterilized filtration and lyophilization, the paclitaxel content in Comparative Example 6 is decreased by about 20% as compared to the paclitaxel content in Example 6.

**Claims**

1.  A method for preparing a drug-containing polymeric micellar nanoparticle composition, comprising:

dissolving a poorly water-soluble drug, a salt of polylactic acid or polylactic acid derivative, and an amphiphilic block copolymer into an organic solvent;
adding an aqueous solution to the resultant mixture in the organic solvent to form micelles; and
adding a lyophilization aid to perform lyophilization,
wherein the carboxylic acid end of the salt of polylactic acid or polylactic acid derivative is bound to an alkali metal ion,
wherein the method requires no separate operation to remove the organic solvent prior to the formation of micelles,
wherein the polylactic acid or polylactic acid derivative is at least one selected from the group consisting of polylactic acid, polylactide, polyglycolide, polymandelic acid, polycaprolactone, polydioxane-2-one, polyaminoacid, polyorthoester, polyanhydride, and copolymers thereof,

wherein the organic solvent is used in an amount of 0.5-15wt% based on the total weight of the composition, and wherein the adding an aqueous solution to the resultant mixture in an organic solvent to form micelles is carried out at 0°C-50°C

2. The method for preparing a drug-containing polymeric micellar nanoparticle composition according to claim 1, wherein the dissolving a poorly water- soluble drug, a salt of polylactic acid or polylactic acid derivative, and an amphiphilic block copolymer into an organic solvent comprises:

dissolving the salt of polylactic acid or polylactic acid derivative, and the amphiphilic block copolymer into an organic solvent; and
dissolving the poorly water-soluble drug into the resultant polymer solution in the organic solvent.

3. The method for preparing a drug-containing polymeric micellar nanoparticle composition according to claim 1, which further comprises adding divalent or trivalent metal ions after forming the micelles.

4. The method for preparing a drug-containing polymeric micellar nanoparticle composition according to claim 1, wherein the adding an aqueous solution to the resultant mixture in an organic solvent to form micelles is carried out at 0°C-40°C.

5. The method for preparing a drug-containing polymeric micellar nanoparticle composition according to claim 1, wherein the poorly water-soluble drug has a solubility of 100 mg/mL or less to water.

6. The method for preparing a drug-containing polymeric micellar nanoparticle composition according to claim 5, wherein the poorly water-soluble drug is a taxane anti-cancer agent, preferably the taxane anti-cancer agent is at least one selected from the group consisting of paclitaxel, docetaxel, 7-epipaclitaxel, t-acetyl paclitaxel, 10-desacetyl-paclitaxel, 10-desacetyl-7-epipaclitaxel, 7-xylosylpaclitaxel, 10-desacetyl-7-glutarylpaclitaxel, 7-N,N-dimethylglycylpaclitaxel, 7-L-alanylpaclitaxel, and a mixture thereof.

7. The method for preparing a drug-containing polymeric micellar nanoparticle composition according to claim 1, wherein the amphiphilic block copolymer comprises a hydrophilic block (A) and a hydrophobic block (B),
the hydrophilic block (A) is at least one selected from the group consisting of polyalkylene glycol, polyethylene glycol, polyvinylpyrrolidone, polyvinyl alcohol, and polyacrylamide, preferably the hydrophilic block (A) has a number average molecular weight of 500-50,000 daltons,
the hydrophobic block (B) is at least one selected from the group consisting of polylactide, polyglycolide, polydioxane-2-one, polycaprolactone, polylactic-co-glycolide, polylactic-*co*-caprolactone, polylactic-*co*-dioxane-2-one, and derivative thereof substituted with fatty acids at hydroxyl end groups, preferably the hydrophobic block (B) has a number average molecular weight of 500-50,000 daltons.

8. The method for preparing a drug-containing polymeric micellar nanoparticle composition according to claim 7, wherein the amphiphilic block copolymer comprises the hydrophilic block (A) and the hydrophobic block (B) in a weight ratio (A:B) of 3:7 to 8:2.

9. The method for preparing a drug-containing polymeric micellar nanoparticle composition according to claim 1, wherein the polylactic acid or polylactic acid derivative has a number average molecular weight of 500-5,000 daltons.

10. The method for preparing a drug-containing polymeric micellar nanoparticle composition according to claim 1, wherein the organic solvent is at least one solvent selected from the group consisting of alcohol, acetone, tetrahydrofuran, acetic acid, acetonitrile, and dioxane and
preferably the alcohol is at least one selected from the group consisting of .methanol, ethanol, propanol, and butanol.

11. The method for preparing a drug-containing polymeric micellar nanoparticle composition according to claim 3, wherein the divalent or trivalent metal ion is selected from the group consisting of calcium ($Ca^{2+}$), magnesium ($Mg^{2+}$), barium ($Ba^{2+}$), manganese ($Mn^{2+}$), nickel ($Ni^{2+}$), copper ($Cu^{2+}$), zinc ($Zn^{2+}$), chrome ($Cr^{3+}$), iron ($Fe^{3+}$), and aluminum ($Al^{3+}$).

12. The method for preparing a drug-containing polymeric micellar nanoparticle composition according to claim 1, wherein the lyophilization aid is sugar, sugar alcohol or a mixture thereof.

**13.** The method for preparing a drug-containing polymeric micellar nanoparticle composition according to claim 1, wherein the poorly water-soluble drug-containing polymeric micellar nanoparticle composition comprises the poorly water-soluble drug in a weight ratio of 0.1-50.0:50.0-99.9 based on the total weight of the polymers; and comprises 0.1-99.9 wt% of the amphiphilic block copolymer and 0.1-99.9 wt% of the salt of polylactic acid or polylactic acid derivative, based on the total weight including the amphiphilic block copolymer and the salt of polylactic acid or polylactic acid derivative.

**14.** The method for preparing a drug-containing polymeric micellar nanoparticle composition according to claim 3, wherein the divalent or trivalent metal ions are used in an amount of 0.5-2.0 equivalents based on the total equivalent of carboxyl end groups of the salt of polylactic acid or polylactic acid derivative.

**Patentansprüche**

**1.** Verfahren zum Herstellen einer arzneimittelhaltigen polymeren mizellaren Nanoteilchenzusammensetzung, das Folgendes umfasst:

Auflösen eines schlecht wasserlöslichen Arzneimittels, eines Salzes von Polymilchsäure oder eines Polymilchsäurederivats und eines amphiphilen Blockcopolymers in einem organischen Lösungsmittel;
Zugeben einer wässrigen Lösung zu der resultierenden Mischung in dem organischen Lösungsmittel, um Mizellen zu bilden; und
Zugeben eines Lyophilisierungshilfsmittels, um eine Lyophilisierung auszuführen,
wobei das Carbonsäureende des Salzes von Polymilchsäure oder des Polymilchsäurederivats an ein Alkalimetallion gebunden ist,
wobei das Verfahren vor der Bildung von Mizellen keinen separaten Arbeitsablauf erfordert, um das organische Lösungsmittel zu entfernen,
wobei die Polymilchsäure oder das Polymilchsäurederivat mindestens eines ist, das ausgewählt ist aus der Gruppe bestehend aus Polymilchsäure, Polylactid, Polyglycolid, Polymandelsäure, Polycaprolacton, Polydioxan-2-on, Polyaminosäure, Polyorthoester, Polyanhydrid und Copolymeren davon,
wobei das organische Lösungsmittel in einer Menge von 0,5 bis 15 Gew.-% basierend auf dem Gesamtgewicht der Zusammensetzung verwendet wird, und
wobei das Zugeben einer wässrigen Lösung zu der resultierenden Mischung in einem organischen Lösungsmittel zur Bildung von Mizellen bei 0 °C bis 50 °C ausgeführt wird.

**2.** Verfahren zum Herstellen einer arzneimittelhaltigen polymeren mizellaren Nanoteilchenzusammensetzung nach Anspruch 1, wobei das Auflösen eines schlecht wasserlöslichen Arzneimittels, eines Salzes von Polymilchsäure oder eines Polymilchsäurederivats und eines amphiphilen Blockcopolymers in einem organischen Lösungsmittel Folgendes umfasst:

Auflösen des Salzes von Polymilchsäure oder eines Polymilchsäurederivats und des amphiphilen Blockcopolymers in einem organischen Lösungsmittel; und
Auflösen des schlecht wasserlöslichen Arzneimittels in der resultierenden Polymerlösung in dem organischen Lösungsmittel.

**3.** Verfahren zum Herstellen einer arzneimittelhaltigen polymeren mizellaren Nanoteilchenzusammensetzung nach Anspruch 1, das ferner das Zugeben von zweiwertigen oder dreiwertigen Metallionen nach dem Bilden der Mizellen umfasst.

**4.** Verfahren zum Herstellen einer arzneimittelhaltigen polymeren mizellaren Nanoteilchenzusammensetzung nach Anspruch 1, wobei das Zugeben einer wässrigen Lösung zu der resultierenden Mischung in einem organischen Lösungsmittel zur Bildung von Mizellen bei 0 °C bis 40 °C ausgeführt wird.

**5.** Verfahren zum Herstellen einer arzneimittelhaltigen polymeren mizellaren Nanoteilchenzusammensetzung nach Anspruch 1, wobei das schlecht wasserlösliche Arzneimittel eine Löslichkeit von 100 mg/ml oder weniger in Wasser aufweist.

**6.** Verfahren zum Herstellen einer arzneimittelhaltigen polymeren mizellaren Nanoteilchenzusammensetzung nach Anspruch 5, wobei das schlecht wasserlösliche Arzneimittel ein Taxan-Antikrebsmittel ist, wobei das Taxan-Antikrebsmittel vorzugsweise mindestens eines ist, das ausgewählt ist aus der Gruppe bestehend aus Paclitaxel, Docetaxel, 7-Epipaclitaxel, t-Acetylpaclitaxel, 10-Desacetylpaclitaxel, 10-Desacetyl-7-epipaclitaxel, 7-Xylosylpaclitaxel, 10-Desacetyl-7-glutarylpaclitaxel, 7-N,N-Dimethylglycylpaclitaxel, 7-L-Alanylpaclitaxel und einer Mischung davon.

7.  Verfahren zum Herstellen einer arzneimittelhaltigen polymeren mizellaren Nanoteilchenzusammensetzung nach Anspruch 1, wobei das amphiphile Blockcopolymer einen hydrophilen Block (A) und einen hydrophoben Block (B) umfasst,
der hydrophile Block (A) mindestens einer ist, der ausgewählt ist aus der Gruppe bestehend aus Polyalkylenglycol, Polyethylenglycol, Polyvinylpyrrolidon, Polyvinylalkohol und Polyacrylamid, wobei der hydrophile Block (A) vorzugsweise ein zahlengemitteltes Molekulargewicht von 500 bis 50.000 Dalton aufweist,
der hydrophobe Block (B) mindestens einer ist, der ausgewählt ist aus der Gruppe bestehend aus Polylactid, Polyglycolid, Polydioxan-2-on, Polycaprolacton, Polymilchsäure-co-glycolid, Polymilchsäure-cocaprolacton, Polymilchsäure-co-dioxan-2-on und einem Derivat davon, das mit Fettsäuren an Hydroxylendgruppen substituiert ist, wobei der hydrophobe Block (B) vorzugsweise ein zahlengemitteltes Molekulargewicht von 500 bis 50.000 Dalton aufweist.

8.  Verfahren zum Herstellen einer arzneimittelhaltigen polymeren mizellaren Nanoteilchenzusammensetzung nach Anspruch 7, wobei das amphiphile Blockcopolymer den hydrophilen Block (A) und den hydrophoben Block (B) in einem Gewichtsverhältnis (A:B) von 3:7 bis 8:2 umfasst.

9.  Verfahren zum Herstellen einer arzneimittelhaltigen polymeren mizellaren Nanoteilchenzusammensetzung nach Anspruch 1, wobei die Polymilchsäure oder das Polymilchsäurederivat ein zahlengemitteltes Molekulargewicht von 500 bis 5.000 Dalton aufweist.

10. Verfahren zum Herstellen einer arzneimittelhaltigen polymeren mizellaren Nanoteilchenzusammensetzung nach Anspruch 1, wobei das organische Lösungsmittel mindestens ein Lösungsmittel ist, das ausgewählt ist aus der Gruppe bestehend aus Alkohol, Aceton, Tetrahydrofuran, Essigsäure, Acetonitril und Dioxan und der Alkohol vorzugsweise mindestens einer ist, der ausgewählt ist aus der Gruppe bestehend aus Methanol, Ethanol, Propanol und Butanol.

11. Verfahren zum Herstellen einer arzneimittelhaltigen polymeren mizellaren Nanoteilchenzusammensetzung nach Anspruch 3, wobei das zweiwertige oder dreiwertige Metallion ausgewählt ist aus der Gruppe bestehend aus Calcium ($Ca^{2+}$), Magnesium ($Mg^{2+}$), Barium ($Ba^{2+}$), Mangan ($Mn^{2+}$), Nickel ($Ni^{2+}$), Kupfer ($Cu^{2+}$), Zink ($Zn^{2+}$), Chrom ($Cr^{3+}$), Eisen ($Fe^{3+}$) und Aluminium ($Al^{3+}$).

12. Verfahren zum Herstellen einer arzneimittelhaltigen polymeren mizellaren Nanoteilchenzusammensetzung nach Anspruch 1, wobei das Lyophilisierungshilfsmittel Zucker, Zuckeralkohol oder eine Mischung davon ist.

13. Verfahren zum Herstellen einer arzneimittelhaltigen polymeren mizellaren Nanoteilchenzusammensetzung nach Anspruch 1, wobei die schlecht wasserlösliche arzneimittelhaltige polymere mizellare Nanoteilchenzusammensetzung das schlecht wasserlösliche Arzneimittel in einem Gewichtsverhältnis von 0,1 bis 50,0 zu 50,0 bis 99,9, bezogen auf das Gesamtgewicht der Polymere umfasst; und 0,1 bis 99,9 Gew.-% des amphiphilen Blockcopolymers und 0,1 bis 99,9 Gew.-% des Salzes von Polymilchsäure oder Polymilchsäurederivats, bezogen auf das Gesamtgewicht, einschließlich des amphiphilen Blockcopolymers und des Salzes von Polymilchsäure oder des Polymilchsäurederivats, umfasst.

14. Verfahren zum Herstellen einer arzneimittelhaltigen polymeren mizellaren Nanoteilchenzusammensetzung nach Anspruch 3, wobei die zweiwertigen oder dreiwertigen Metallionen in einer Menge von 0,5 bis 2,0 Äquivalenten basierend auf dem Gesamtäquivalent von Carboxylendgruppen des Salzes von Polymilchsäure oder des Polymilchsäurederivats verwendet werden.

## Revendications

1.  Procédé pour la préparation d'une composition de nanoparticules micellaires polymères contenant un médicament, comprenant :

la dissolution d'un médicament faiblement soluble dans l'eau, d'un sel d'un acide polylactique ou d'un dérivé d'un acide polylactique, et d'un copolymère séquencé amphiphile dans un solvant organique ;

l'ajout d'une solution aqueuse au mélange résultant dans le solvant organique afin de former des micelles ; et l'ajout d'un adjuvant de lyophilisation afin d'effectuer la lyophilisation,

dans lequel l'extrémité acide carboxylique du sel d'acide polylactique ou du dérivé d'acide polylactique est liée à un ion de métal alcalin,

dans lequel le procédé ne nécessite aucune opération de séparation pour éliminer le solvant organique avant la formation des micelles,

dans lequel l'acide polylactique ou le dérivé d'acide polylactique est au moins un choisi dans le groupe consistant en un acide polylactique, un polylactide, un polyglycolide, un acide polymandélique, une polycaprolactone, une polydioxane-2-one, un polyaminoacide, un polyorthoester, un polyanhydride, et des copolymères de ceux-ci,

dans lequel le solvant organique est utilisé en une quantité de 0,5 à 15 % en poids sur la base du poids total de la composition, et

dans lequel l'ajout d'une solution aqueuse au mélange résultant dans le solvant organique afin de former des micelles est mis en oeuvre à 0 °C à 50 °C.

2. Procédé pour la préparation d'une composition de nanoparticules micellaires polymères contenant un médicament selon la revendication 1, dans lequel la dissolution d'un médicament faiblement soluble dans l'eau, d'un sel d'un acide polylactique ou d'un dérivé d'un acide polylactique, et d'un copolymère séquencé amphiphile dans un solvant organique comprend :

la dissolution du sel d'un acide polylactique ou d'un dérivé d'un acide polylactique, et du copolymère séquencé amphiphile dans un solvant organique ; et

la dissolution du médicament faiblement soluble dans l'eau dans la solution polymère résultante dans le solvant organique.

3. Procédé pour la préparation d'une composition de nanoparticules micellaires polymères contenant un médicament selon la revendication 1, qui comprend en outre l'ajout d'ions métalliques divalents ou trivalents après la formation des micelles.

4. Procédé pour la préparation d'une composition de nanoparticules micellaires polymères contenant un médicament selon la revendication 1, dans lequel l'ajout d'une solution aqueuse au mélange résultant dans le solvant organique afin de former des micelles est mis en oeuvre à 0 °C à 40 °C.

5. Procédé pour la préparation d'une composition de nanoparticules micellaires polymères contenant un médicament selon la revendication 1, dans lequel le médicament faiblement soluble dans l'eau a une solubilité de 100 mg/mL ou moins dans l'eau.

6. Procédé pour la préparation d'une composition de nanoparticules micellaires polymères contenant un médicament selon la revendication 5, dans lequel le médicament faiblement soluble dans l'eau est un agent anti-cancéreux de type taxane, l'agent anti-cancéreux de type taxane est de préférence au moins un choisi dans le groupe consistant en le paclitaxel, le docétaxel, le 7-épipaclitaxel, le t-acétyl paclitaxel, le 10-désacétyl-paclitaxel, le 10-désacétyl-7-épipaclitaxel, le 7-xylosyl paclitaxel, le 10-désacétyl-7-glutaryl paclitaxel, le 7-N,N-diméthylglycyl paclitaxel, le 7-L-alanyl paclitaxel, et un mélange de ceux-ci.

7. Procédé pour la préparation d'une composition de nanoparticules micellaires polymères contenant un médicament selon la revendication 1, dans lequel le copolymère séquencé amphiphile comprend une séquence hydrophile (A) et une séquence hydrophobe (B),

la séquence hydrophile (A) est au moins un choisi dans le groupe consistant en un polyalkylène glycol, un polyéthylène glycol, une polyvinylpyrrolidone, un alcool polyvinylique, et un polyacrylamide, la séquence hydrophile (A) a de préférence une masse moléculaire moyenne en nombre de 500 à 50 000 daltons,

la séquence hydrophobe (B) est au moins un choisi dans le groupe consistant en un polylactide, un polyglycolide, une polydioxane-2-one, une polycaprolactone, un poly(acide lactique-co-glycolide), un poly(acide lactique-co-caprolactone), un poly(acide lactique-co-dioxane-2-one), et des dérivés de ceux-ci substitués par des acides gras au niveau des groupes terminaux hydroxyle, la séquence hydrophobe (B) a de préférence une masse moléculaire moyenne en nombre de 500 à 50 000 daltons.

8. Procédé pour la préparation d'une composition de nanoparticules micellaires polymères contenant un médicament selon la revendication 7, dans lequel le copolymère séquencé amphiphile comprend la séquence hydrophile (A) et la séquence hydrophobe (B) selon un rapport pondéral (A:B) de 3:7 à 8:2.

9. Procédé pour la préparation d'une composition de nanoparticules micellaires polymères contenant un médicament selon la revendication 1, dans lequel l'acide polylactique ou le dérivé d'acide polylactique a une masse moléculaire moyenne en nombre de 500 à 5 000 daltons.

10. Procédé pour la préparation d'une composition de nanoparticules micellaires polymères contenant un médicament selon la revendication 1, dans lequel le solvant organique est au moins un solvant choisi dans le groupe consistant en un alcool, l'acétone, le tétrahydrofuranne, l'acide acétique, l'acétonitrile, et le dioxane et l'alcool est de préférence au moins un alcool choisi dans le groupe consistant en le méthanol, l'éthanol, le propanol, et le butanol.

11. Procédé pour la préparation d'une composition de nanoparticules micellaires polymères contenant un médicament selon la revendication 3, dans lequel l'ion métallique divalent ou trivalent est choisi dans le groupe consistant en le calcium ($Ca^{2+}$), le magnésium ($Mg^{2+}$), le barium ($Ba^{2+}$), le manganèse ($Mn^{2+}$), le nickel ($Ni^{2+}$), le cuivre ($Cu^{2+}$), le zinc ($Zn^{2+}$), le chrome ($Cr^{3+}$), le fer ($Fe^{3+}$), et l'aluminium ($Al^{3+}$).

12. Procédé pour la préparation d'une composition de nanoparticules micellaires polymères contenant un médicament selon la revendication 1, dans lequel l'adjuvant de lyophilisation est un sucre, un alcool de sucre ou un mélange de ceux-ci.

13. Procédé pour la préparation d'une composition de nanoparticules micellaires polymères contenant un médicament selon la revendication 1, dans lequel la composition de nanoparticules micellaires polymères contenant un médicament faiblement soluble dans l'eau comprend le médicament faiblement soluble dans l'eau selon un rapport pondéral de 0,1-50,0:50,0-99,9 sur la base du poids total des polymères ; et comprend de 0,1 à 99,9 % en poids du copolymère séquencé amphiphile et de 0,1 à 99,9 % en poids du sel d'acide polylactique ou du dérivé d'acide polylactique, sur la base du poids total comprenant le copolymère séquencé amphiphile et le sel d'acide polylactique ou du dérivé d'acide polylactique.

14. Procédé pour la préparation d'une composition de nanoparticules micellaires polymères contenant un médicament selon la revendication 3, dans lequel les ions métalliques divalents ou trivalents sont utilisés en une quantité de 0,5 à 2,0 équivalents sur la base du total d'équivalents des groupes terminaux carboxyle du sel d'acide polylactique ou du dérivé d'acide polylactique.

**EP 2 376 062 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03033592 A